(19) Europäisches Patentamt
European Patent Office
Office européen des brevets

(11) Veröffentlichungsnummer: **0 051 558**
**B1**

# (12) EUROPÄISCHE PATENTSCHRIFT

(45) Veröffentlichungstag der Patentschrift: **28.03.84**

(51) Int. Cl.³: **C 07 D 307/935,** A 61 K 31/34 // C07F7/18

(21) Anmeldenummer: **81730114.6**

(22) Anmeldetag: **29.10.81**

(54) **Prostacyclinderivate, Verfahren zu ihrer Herstellung und ihre Verwendung als Arzneimittel.**

(30) Priorität: **31.10.80 DE 3041601**

(43) Veröffentlichungstag der Anmeldung: **12.05.82 Patentblatt 82/19**

(45) Bekanntmachung des Hinweises auf die Patenterteilung: **28.03.84 Patentblatt 84/13**

(84) Benannte Vertragsstaaten: **AT BE CH DE FR GB IT LI LU NL SE**

(56) Entgegenhaltungen: **DE - A - 2 753 244**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Skuballa, Werner, Dr., Olwenstrasse 25, D-1000 Berlin 28 (DE)**
Erfinder: **Vorbrüggen, Helmut, Prof., Wilkestrasse 7, D-1000 Berlin 27 (DE)**
Erfinder: **Radüchel, Bernd, Dr., Gollanczstrasse 132, D-1000 Berlin 28 (DE)**
Erfinder: **Loge, Olaf, Dr., Bekassinen Weg 27, D-1000 Berlin 27 (DE)**
Erfinder: **Vischer, Peter, Dr., Am Schweizerhof 11, D-1000 Berlin 37 (DE)**

**EP 0 051 558 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Prostacyclinderivate, Verfahren zu ihrer Herstellung und ihre Verwendung als Arzneimittel

Die Erfindung betrifft neue Prostacyclinderivate, Verfahren zu ihrer Herstellung sowie ihre Verwendung als Arzneimittel.

Prostacyclin ($PGI_2$), einer der Hauptfaktoren bei der Blutplättchenaggregation, wirkt dilatierend auf verschiedene Blutgefässe (Science 196, 1072) und könnte daher als Mittel zur Blutdrucksenkung in Betracht kommen. $PGI_2$ besitzt jedoch nicht die für ein Arzneimittel notwendige Stabilität. So beträgt seine Halbwertszeit bei physiologischen pH-Werten und bei Raumtemperatur nur wenige Minuten.

In der DE-OS 27 53 244 werden Prostacyclinderivate, die durch eine Nitrilgruppe an der Enolätherdoppelbindung stabilisiert sind, beschrieben.

Es wurde nun gefunden, dass durch Ersatz der 1-Carboxylgruppe in den 5-Cyano-Prostacyclinen durch eine Hydroxymethylgruppe eine längere Wirkungsdauer und grössere Selektivität erzielt werden kann.

Die erfindungsgemässen Verbindungen wirken vasodilatierend, blutdrucksenkend und bronchodilatorisch. Sie sind ausserdem zur Inhibierung der Thrombozytenaggregation und der Magensäuresekretion geeignet.

Die Erfindung betrifft daher Prostacyclinderivate der allgemeinen Formel I

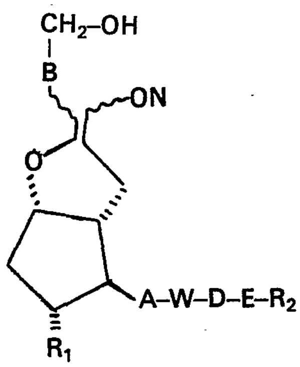

(I)

worin

B eine geradkettige oder verzweigtkettige Alkylengruppe mit 1–5 C-Atomen,

A eine $-CH_2-CH_2-$, trans-CH=CH- oder $-C\equiv C-$Gruppe,

W eine freie oder funktionell abgewandelte Hydroxymethylengruppe oder freie oder funktionell abgewandelte $-\overset{CH_3}{\underset{OH}{C}}-$Gruppe, wobei die OH-Gruppe α- oder β-ständig sein kann,

D und E gemeinsam eine direkte Bindung oder

D eine geradkettige oder verzweigte gesättigte Alkylengruppe mit 1–5 C-Atomen, die gegebenenfalls durch Fluoratome substituiert sein kann,

E Sauerstoff, eine $-C\equiv C-$Bindung oder eine direkte Bindung,

$R_2$ eine Alkylgruppe mit 1–7 C-Atomen oder eine Phenylgruppe und

$R_1$ eine freie oder funktionell abgewandelte Hydroxygruppe bedeuten.

Die Hydroxygruppen $R_1$ und in W können funktionell abgewandelt sein, beispielsweise durch Verätherung oder Veresterung, wobei die freien oder abgewandelten Hydroxygruppen in W α- oder β-ständig sein können, wobei freie Hydroxygruppen bevorzugt sind. Als Äther- und Acylreste kommen die dem Fachmann bekannten Reste in Betracht. Bevorzugt sind leicht abspaltbare Ätherreste wie beispielsweise der Tetrahydropyranyl-, Tetrahydrofuranyl-, α-Äthoxyäthyl-, Trimethylsilyl-, Dimethyl-tert.-butylsilyl- und Tribenzylsilylrest. Als Acylreste kommen $C_1$–$C_4$-Alkanoylreste wie beispielsweise Acetyl, Propionyl, Butyryl oder Benzoyl in Frage.

Als $R_2$ kommen gerad- und verzweigkettige, gesättigte und ungesättigte Alkylreste mit 1–7 C-Atomen oder die Phenylgruppe in Frage.

Als Alkylengruppe D kommen geradkettige oder verzweigtkettige gesättigte Alkylenreste mit 1–5 C-Atomen in Frage, die gegebenenfalls durch Fluoratome substituiert sein können. Beispielsweise seien genannt: Methylen, Fluormethylen, Äthylen, 1,2-Propylen, Äthyläthylen, Trimethylen, Tetramethylen, Pentamethylen, 1-Methyl-tetramethylen, 1-Methyl-trimethylen, 2-Methyltrimethylen, 2-Methyl-tetramethylen.

Als Alkylengruppe B kommen geradkettige oder verzweigtkettige Alkylenreste mit 1–5 C-Atomen in Frage. Beispielsweise seien genannt: Methylen, Äthylen, Trimethylen, Tetramethylen und Pentamethylen.

Die Erfindung betrifft ferner ein Verfahren zur Herstellung der erfindungsgemässen Prostacyclinderivate der allgemeinen Formel I, dadurch gekennzeichnet, dass man eine Verbindung der allgemeinen Formel II

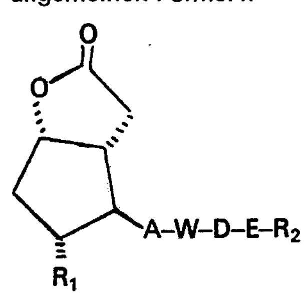

(II)

worin $R_1$, $R_2$, A, W, D, E die oben angegebenen Bedeutungen aufweisen, gegebenenfalls nach Schutz anwesender freier Hydroxygruppen nach Umsetzung mit einem aus dem Nitril der allgemeinen Formel III und Lithiumdiisopropylamid hergestellten Carbanion, wobei $R_3$ einen leicht abspaltbaren Ätherrest

$$N\equiv C-CH_2-B-CH_2OR_3 \qquad (III)$$

bedeutet und B die oben angegebene Bedeutung besitzt, einer Säurebehandlung unterwirft.

Gegebenenfalls kann man anschliessend in den so erhaltenen Verfahrensprodukten in beliebiger Reihenfolge, Isomere trennen, geschützte Hydroxygruppen freisetzen und/oder freie Hydroxygruppen verestern oder veräthern.

Als Ätherreste $R_3$ in der Verbindung der allgemeinen Formel III kommen die dem Fachmann geläufigen Reste in Betracht. Bevorzugt sind leicht abspaltbare Ätherreste wie beispielsweise Dimethyl-tert.-butyl-silyl-, Trimethylsilyl-, Tribenzylsilyl-, Tetrahydropyranyl-, Tetrahydrofuranyl- und α-Äthoxyäthyl, um nur einige zu nennen.

Die Umsetzung der Verbindung der allgemeinen Formel II (Herstellung analog DE-OS 28 45 770) mit der metallorganischen Verbindung der allgemeinen Formel III, die man aus dem entsprechenden Nitril mit Lithiumdiisopropylamid in Äther-Tetrahydrofurangemischen in Gegenwart von Hexamethylphosphorsäuretriamid herstellt, wird bei Temperaturen von 0 bis −100 °C, vorzugsweise bei −60 bis −78 °C, in einem für metallorganische Reaktionen geeigneten Lösungsmittel oder Lösungsmittelgemisch, vorzugsweise Diäthyläther oder Tetrahydrofuran, vorgenommen. Zur Wasserabspaltung behandelt man das Rohprodukt der metallorganischen Umsetzung mit einer katalytischen Menge einer Säure in einem mit Wasser nicht mischbaren Lösungsmittel wie beispielsweise Toluol, Benzol, Methylenchlorid, Chloroform, Tetrachlorkohlenstoff oder Diäthyläther vorzugsweise in Toluol oder Diäthyläther bei Temperaturen zwischen −20 und 100 °C, vorzugsweise 0 bis 30 °C. Als Säuren kommen beispielsweise in Frage p-Toluolsulfonsäure, Schwefelsäure, Salzsäure oder Bortrifluorid.

Die funktionelle Abwandlung der freien OH-Gruppen erfolgt nach den dem Fachmann bekannten Methoden. Zur Einführung der Ätherschutzgruppe wird beispielsweise mit Dihydropyran in Methylenchlorid, Benzol oder Chloroform unter Verwendung eines sauren Katalysators, wie zum Beispiel $POCl_3$, p-Toluolsulfonsäure oder wasserfreier Mineralsäuren umgesetzt. Das Dihydropyran wird im Überschuss angewandt, vorzugsweise in der 2- bis 10-fachen Menge des theoretischen Bedarfs. Die Umsetzung ist normalerweise bei 0 bis 30 °C nach 15 bis 30 Minuten beendet.

Die Einführung der Acylschutzgruppe erfolgt, indem man eine Verbindung der allgemeinen Formel I in an sich bekannter Weise mit einem Carbonsäurederivat, wie zum Beispiel Säurechlorid oder Säureanhydrid in Gegenwart einer tertiären Aminbase, wie z.B. Pyridin oder Dimethylaminopyridin umsetzt.

Die Freisetzung einer funktionell abgewandelten OH-Gruppe zu den Verbindungen der allgemeinen Formel I erfolgt nach bekannten Methoden. Beispielsweise wird die Abspaltung der Ätherschutzgruppe in einer wässrigen Lösung einer organischen Säure, wie zum Beispiel Essigsäure oder Propionsäure oder in einer wässrigen Lösung einer anorganischen Säure, wie z.B. Salzsäure, durchgeführt. Zur Verbesserung der Löslichkeit wird zweckmässigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20 und 80 °C durchgeführt.

Die Abspaltung der Silylätherschutzgruppe erfolgt beispielsweise mit Tetrabutylammmoniumfluorid oder mit KF in Gegenwart eines Kronenäthers. Als Lösungsmittel sind beispielsweise geeignet Tetrahydrofuran, Diäthyläther, Dioxan oder Methylenchlorid. Die Abspaltung wird vorzugsweise bei Temperaturen zwischen 0 und 80 °C durchgeführt.

Die Verseifung der Acylgruppe erfolgt beispielsweise mit Alkali- oder Erdalkali-carbonaten oder -hydroxyden in einem Alkohol oder in der wässrigen Lösung eines Alkohols. Als Alkohole kommen aliphatische Alkohole in Betracht, wie zum Beispiel Methanol, Äthanol oder Butanol, vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei −10 bis 70 °C, vorzugsweise bei 25 °C.

Die so hergestellten Nitrile der allgemeinen Formel I stellen in Bezug auf die der Cyangruppe benachbarte Doppelbindung Isomerengemische dar, die durch übliche Trennmethoden, wie beispielsweise Säulenchromatographie oder Schichtchromatographie getrennt werden können.

Die für das Verfahren verwendeten Nitrile der allgemeinen Formel III können beispielsweise aus 1,5-Alkandiolen durch selektive Silylierung, Tosylierung und anschliessende Umsetzung mit Natrium- oder Kaliumcyanid hergestellt werden.

Die Verbindungen dieser Erfindung wirken blutdrucksenkend und bronchodilatorisch. Sie sind weiterhin geeignet zur Hemmung der Thrombozyten-Aggregation. Folglich stellen die neuen Prostacyclin-Derivate der allgemeinen Formel I wertvolle pharmazeutische Wirkstoffe dar. Darüber hinaus weisen sie bei ähnlichem Wirkungsspektrum, verglichen mit entsprechenden Prostaglandinen, eine höhere Spezifität und vor allem eine wesentlich längere Wirksamkeit auf. Im Vergleich zu $PGI_2$ zeichnen sie sich durch grössere Stabilität aus. Die hohe Gewebsspezifität der neuen Prostaglandine zeigt sich bei der Untersuchung an glattmuskulären Organen, wie z.B. am Meerschweinchenileum oder an der isolierten Kaninchentrachea, wo eine wesentlich geringere Stimulation zu beobachten ist als bei der Applikation natürlicher Prostaglandine vom E-, A- oder F-Typ.

Die neuen Prostaglandin-Analoga besitzen die für Prostacycline typischen Eigenschaften, wie z.B. Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdrukkes ohne zugleich Schlagvolumen und koronare Durchblutung zu senken; Behandlung von Schlaganfall, Prophylaxe und Therapie koronarer Herzerkrankungen, Koronarer Thrombose, des Herzinfarkts, peripherer Arterienerkrankungen, Arteriosklerose und Thrombose, Therapie des Schocks, Inhibierung der Bronchokonstriktion, Inhibierung der Magensäuresekretion und Zytoprotektion der Magen- und Darmschleimhaut; Zytoprotektion am Pankreas, antiallergische Eigenschaften, Senkung des pulmonaren vaskulären Widerstandes und des pulmonaren Blutdrukkes, Förderung der Nierendurchblutung, Anwendung an Stelle von Heparin oder als Adjuvans bei der Dialyse der Hämofiltration, Konservierung von Blutplasmakonserven, besonders vor Blutplättchenkonserven, Inhibierung von Geburtswehen, Behandlung von Schwangerschaftstoxikose, Erhöhung der zerebralen Durchblutung etc. Ausserdem besitzen die neuen Prostaglandine antiprolifertive Eigenschaften.

Die Dosis der Verbindungen ist 1–1500 µg/kg/Tag, wenn sie am menschlichen Patienten verabreicht werden. Die Einheitsdosis für den pharmazeutischen akzeptablen Träger beträgt 0,01–100 mg.

Bei intravenöser Injektion an wachen, hypertonen Ratten in Dosen von 5, 20 und 100 µg/kg Körpergewicht zeigen die erfindungsgemässen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intensiver Injektion an narkotisierten Kaninchen zeigen die erfindungsgemässen Verbindungen in Vergleich zu $PGE_2$ und $PGA_2$ eine stärkere und erheblich länger anhaltende Blutdrucksenkung, ohne dass andere glattmuskuläre Organe oder Organfunktionen beeinflusst werden.

Für die parenterale Verabreichung werden sterile, injizierbare, wässrige oder ölige Lösungen benutzt. Für die orale Applikation sind beispielsweise Tabletten, Dragees oder Kapseln geeignet.

Die Erfindung betrifft somit auch Arzneimittel auf Basis der Verbindungen der allgemeinen Formel I und üblicher Hilfs- und Trägerstoffe.

Die erfindungsgemässen Wirkstoffe sollen in Verbindung mit den in der Galenik bekannten und üblichen Hilfsstoffen z.B. zur Herstellung von Blutdrucksenkern dienen.

Beispiel 1

5-Cyano-2-descarboxy-2-hydroxymethyl-prostacyclin

5,1 ml Diisopropylamin versetzt man bei −25 °C innerhalb von 15 Minuten mit 23,5 ml einer 1,53 molaren Lösung von Butyllithium in Hexan und rührt 1 Stunde bei −25 °C. Anschliessend versetzt man mit 6,3 ml Hexamethylphosphorsäuretriamid und tropft in diese Mischung bei −70 °C innerhalb von 30 Minuten eine Lösung aus 8,2 g 6-(Dimethyl-tert.-butylsilyloxy)-hexannitril in 5 ml Tetrahydrofuran. Man rührt 20 Minuten bei −70 °C, fügt eine Lösung von 4,7 g (1S, 5R, 6R, 7R)-6- [(E) -(3S) -3-Benzoyloxy-1-octenyl]-7-benzoyloxy-2-oxabicyclo [3.3.0] -octan-3-on in 25 ml Äther und 30 ml Tetrahydrofuran zu, rührt 20 Minuten bei −70 °C und säuert die Reaktionsmischung durch Eingiessen in eine 10%ige Citronensäurelösung auf einen pH-Wert von 5 an. Man extrahiert dreimal mit je 200 ml Äther, schüttelt die organische Phase zweimal mit Wasser, trocknet über Natriumsulfat und filtriert den Eindampfrückstand in Essigester über Kieselgel. Dabei erhält man 3,3 g des Reaktionsprodukts der metallorganischen Umsetzung als 11,15-Dihydroxyverbindung.

Zur Wasserabspaltung löst man das Reaktionsprodukt der vorstehend beschriebenen Umsetzung in 150 ml abs. Diäthyläther, fügt 90 ml einer verdünnten ätherischen Bortrifluorid-Lösung (Herstellung: Man verdünnt 0,9 ml 45%ige Bortrifluorid-ätherat-Lösung mit 81 ml abs. Äther) zu und rührt 1 Stunde bei Raumtemperatur unter Argon. Anschliessend giesst man auf 5%ige Natriumbicarbonatlösung, wäscht mit Wasser neutral, trocknet über Natriumsulfat und dampft im Vakuum ein. Nach Chromatographie des Rückstandes an Kieselgel erhält man zunächst 1,2 g (5E)-5-Cyano-2-descarboxy-2- (dimethyl-tert.-butylsilyloxymethyl)-prostacyclin und als polarere Komponente 0,98 g des entsprechenden isomeren (5Z) -5-Cyano-2-descarboxy-2- (dimethyl-tert.-butyloxymethyl)-prostacyclins.

Zur Silylätherspaltung rührt man die 1,2 g der (5E)-konfigurierten Verbindung 18 Stunden bei Raumtemperatur mit 40 ml einer Mischung aus Eisessig/Wasser/Tetrahydrofuran (65 + 35 + 10). Man dampft im Vakuum ein und chromatographiert den Rückstand an Kieselgel. Mit Methylenchlorid/Isopropanol (9+1) erhält man 740 mg der Titelverbindung als farbloses Öl.

IR: 3610, 3400 (breit), 2960, 2930, 2860, 2200, 1650, 1600, 970/cm.

Das Ausgangsmaterial für die obige Titelverbindung wird wie folgt hergestellt:

1a) (1S,5R,6R,7R)-6-[(E)-(3S) -3-Benzoyloxy- 1-octenyl]- 7-benzoyloxy- 2-oxabicyclo [3.3.0] octan-3-on.

Zu einer Lösung von 3,1 g (1S, 5R, 6R, 7R)-6-[(E)- (3S) -3-Hydroxy-1-octenyl]-7-benzoyloxy-2-oxabicyclo[3.3.0]octan-3-on in 15 ml Pyridin fügt man bei 0 °C 1,9 ml Benzoylchlorid und rührt 18 Stunden bei Raumtemperatur. Anschliessend versetzt man mit 1,2 ml Wasser, rührt 2 Stunden, verdünnt mit 300 ml Äther, schüttelt einmal mit 50 ml Wasser, zweimal mit 10%iger Schwefelsäure, einmal mit 5%iger Natriumbicarbonatlösung und dreimal mit Wasser. Man trocknet über Magnesiumsulfat, dampft im Vakuum ein und filtriert den Rückstand über Kieselgel. Mit Äther/Hexan (8+2) erhält man 3,9 g des Dibenzoats als farbloses Öl.

IR: 2960, 2925, 1770, 1715, 1602, 1585, 1270, 969/cm.

1b) 6-(Dimethyl- tert.- butylsilyloxy)-hexannitril

Zu einer Lösung von 62,5 g 1,5-Pentandiol und 102 g Imidazol in 100 ml Dimethylformamid fügt man bei Eisbadtemperatur 90,5 g Dimethyl-tert.-butylsilylchlorid und rührt 16 Stunden bei 0 °C. Anschliessend giesst man auf 900 ml Wasser, extrahiert dreimal mit je 500 ml einer Mischung aus Hexan/Äther (1 + 1), wäscht den organischen Extrakt mit Wasser neutral und trocknet über Magnesiumsulfat. Man engt im Vakuum ein und destilliert den Rückstand im Vakuum bei 0,6 Torr. Dabei erhält man bei 76–80 °C 55 g des Monosilyläthers als farblose Flüssigkeit.

Zur Tosylatbildung löst man in 185 ml Pyridin und fügt bei Eisbadtemperatur 74 g p-Toluolsulfonsäurechlorid zu. Man rührt 16 Stunden bei Raumtemperatur, versetzt mit 10 ml Wasser, rührt 3 Stunden, verdünnt mit 1,3 Ltr. Äther, schüttelt zweimal mit 10%iger Schwefelsäure, einmal mit 5%iger Natriumbicarbonatlösung und dreimal mit Wasser. Man trocknet über Magnesiumsulfat und dampft im Vakuum ein. Dabei erhält man 79 g des Tosylats, das man in 185 ml Dimethylsulfoxid löst, mit 22 g Natriumcyanid versetzt und 18 Stunden bei 80 °C unter Argon rührt. Anschliessend versetzt man mit 700 ml Wasser, extrahiert dreimal mit je 400 ml einer Mischung aus Äther/Hexan (1 + 1), wäscht den organischen Extrakt mit Wasser neutral und trocknet über Magnesiumsulfat. Man engt im Vakuum ein und destilliert den Rückstand im Vakuum bei 0,01 Torr. Dabei erhält man bei 75–77 °C 43 g der Titelverbindung als farblose Flüssigkeit.

IR: 2930, 2855, 2242, 1250, 1095, 830/cm.

Beispiel 2

5-Cyano-2-descarboxy-2-hydroxymethyl-16-phenoxy-17,18,19,20-tetranor-prostacyclin

2,95 ml Diisopropylamin versetzt man bei −25 °C innerhalb von 15 Minuten mit 13,95 ml einer 1,55 ml molaren Lösung von Butyllithium in Hexan und rührt 1 Stunde bei −25 °C. Anschliessend versetzt man mit 3,7 ml Hexamethylphosphorsäuretriamid und tropft in diese Mischung bei −70 °C innerhalb von 30 Minuten eine Lösung aus 4,8 g 6-(Dimethyl-tert.-butylsilyloxy)-hexannitril in 4 ml Tetrahydrofuran. Nach 20 Minuten fügt man eine Lösung aus 3 g (1S,5R,6R,7R) -6- [(E)-(3R) -3-Benzoyloxy-4-phenoxy -1- butenyl] -7- benzoyloxy-2-oxabicyclo[3.3.0]octan-3-on in 15 ml Tetrahydrofuran und 15 ml Äther zu, rührt 30 min bei −70 °C und säuert die Reaktionsmischung durch Eingiessen in eine 10%ige Citronensäurelösung auf einen pH-Wert von 5 an. Man extrahiert mit Äther, wäscht die organische Phase mit Wasser neutral, trocknet über Magnesiumsulfat und dampft im Vakuum ein. Nach Chromatographie des Rückstandes an Kieselgel erhält man 2,3 g des Reaktionsproduktes der metallorganischen Umsetzung als 11,15-Diol.

Zur Wasserabspaltung löst man das Reaktionsprodukt der vorstehend beschriebenen Umsetzung in 120 ml abs. Diäthyläther, fügt 60 ml einer verdünnten ätherischen Bortrifluorid-Lösung (Herstellung: Beispiel 1) zu und rührt 1 Stunde bei Raumtemperatur. Anschliessend giesst man auf 5%ige Natriumbicarbonatlösung, wäscht mit Wasser neutral, trocknet über Magnesiumsulfat und dampft im Vakuum ein. Nach Chromatographie des Rückstandes an Kieselgel erhält man zunächst 0,9 g (5E)-5-Cyano-2-descarboxy-2-(dimethyl- tert.- butylsilyloxymethyl)-16-phenoxy-17,18,19,20-tetranor-prostacyclin und als polarere Komponente 0,78 g des entsprechenden isomeren (5Z)-5-Cyano-2-descarboxy-2- (dimethyl-tert.- butylsilyloxymethyl) -16-phenoxy-17, 18, 19, 20-tetranor-prostacyclins.

Zur Silylätherspaltung rührt man 0,9 g der (5E)-konfigurierten Verbindung 18 Stunden bei Raumtemperatur mit 36 ml einer Mischung aus Eisessig/Wasser/Tetrahydrofuran (65 + 35 + 10) und dampft anschliessend im Vakuum ein. Durch Chromatographie des Rückstandes an Kieselgel erhält man mit Methylenchlorid/Isopropanol (9 + 1) 620 mg der Titelverbindung als farbloses Öl.

IR: 3600, 3400, 2930, 2860, 2198, 1650, 1599, 1586, 970 cm.

2a) (1S, 5R, 6R,7R)-6-[(E)-(3R)-3-Benzoyloxy-4-phenoxy-1-butenyl] -7-benzoyloxy-2-oxabicyclo[3.3.]octan-3-on

In Analogie zu Beispiel 1a erhält man aus 5 g (1S,5R,6R,7R) -6- [(E)- (3R) -3-hydroxy-4-phenoxy-1- butenyl] -7-benzoyloxy-2-oxabicyclo[3.3.0]octan-3-on, 22 ml Pyridin und 2,84 g Benzoylchlorid 6,1 g der Titelverbindung als farbloses Öl.

IR: 2940, 1770, 1714, 1599, 1586, 1270, 970/cm.

Beispiel 3

5-Cyano-2-descarboxy-2-hydroxymethyl- 16, 16-dimethyl-prostacyclin

In Analogie zu Beispiel 1 erhält man aus 2,1 g (1S,5R,6R,7R)-6-[(E)-(3R)-3-Benzoyloxy-4,4-dimethyl-1-octenyl] -7-benzoyloxy-2-oxabicyclo[3.3.0]octan-3-on 420 mg der Titelverbindung als farbloses Öl.

IR: 3600, 3400, 2962, 2935, 2860, 2200, 1650, 1600, 972/cm.

Das Ausgangsmaterial für die obige Titelverbindung wird wie folgt hergestellt:

3a) (1S,5R,6R,7R) -6-[(E)-(3R) -3-Benzoyloxy-4,4-dimethyl-1-octenyl] -7- benzoyloxy-2-oxabicyclo[3.3.0]octan-3-on

In Analogie zu Beispiel 1a erhält man aus 1,6 g (1S,5R,6R,7R) -6-[(E)-(3R)-3-Hydroxy-4,4-dimethyl-1-octenyl] -7-benzoyloxy-2-oxybicyclo [3.3.0]octan-3-on, 8 ml Pyridin und 1 ml Benzoylchlorid 2 g des Dibenzoats als farbloses Öl.

IR: 2962, 2930, 1770, 1715, 1600, 1588, 1270, 970/cm.

Beispiel 4

5-Cyano-2-descarboxy-2-hydroxymethyl-15-methyl-prostacyclin

In Analogie zu Beispiel 1 erhält man aus 0,5 g

(1S,5R,6R,7R)-6- [(E)- (3S) -3-Hydroxy-3-methyl-1-octenyl] -7- benzoyloxy-2-oxabicyclo [3.3.0]octan-3-on und 850 mg 6-Dimethyl-tert.-butylsilyloxy-hexannitril 85 mg der Titelverbindung als farbloses Öl.

IR: 3600, 3400 (breit), 2965, 2935, 2863, 2200, 1650, 1602, 972/cm.

Beispiel 5

5-Cyano-2-descarboxy-2-hydroxymethyl-16-methyl-prostacyclin

In Analogie zu Beispiel 1 erhält man aus 2,03 g (1S, 5R, 6R, 7R)-6-[(E)-(3S, 4RS)-3-Benzoyloxy-4-methyl- 1-octenyl]- 7-benzoyloxy-2-oxabicyclo [3.3.0] octan-3-on 425 mg der Titelverbindung als farbloses Öl.

IR: 3610, 3420, 2960, 2935, 2863, 2200, 1650, 1600, 970/cm.

Das Ausgangsmaterial für die obige Titelverbindung wird wie folgt hergestellt:

5a) (1S, 5R, 6R, 7R)- 6-[(E)- (3S, 4RS)-3-Benzoyloxy- 4-methyl- 1-octenyl]- 7-benzoyloxy-2-oxabicyclo [3.3.0]octan-3-on

In Analogie zu Beispiel 1a erhält man aus 1,7 g (1S, 5R, 6R, 7R)-6-[(E) -(3S,4RS)-3-Hydroxy-4-methyl-1- octenyl] -7- benzoyloxy-2-oxabicyclo [3.3.0]octan-3-on, 8 ml Pyridin und 1 ml Benzoylchlorid 2,06 g des Dibenzoats als farbloses Öl.

IR: 2960, 2935, 1771, 1715, 1600, 1589, 1270, 972/cm.

Beispiel 6

5-Cyano- 2-descarboxy-2-hydroxymethyl-16- fluor-prostacyclin

In Analogie zu Beispiel 1 erhält man aus 2 g (1S, 5R, 6R, 7R)-6-[(E)-(®,4RS)-3-Benzoyloxy-4-fluor-1-octenyl] -7-benzoyloxy -2-oxabicyclo [3.3.0] octan -3-on 630 mg der Titelverbindung als Öl.

IR: 3630, 3410, 2958, 2936, 2860, 2202, 1650, 972/cm.

Das Ausgangsmaterial für die obige Titelverbindung wird wie folgt hergestellt:

6a) (1S,5R,6R,7R)-6- [(E)-(3R,4RS)-3-Benzoyloxy-4-fluor-1-octenyl] -7- benzoyloxy-2-oxabicyclo [3. 3.0]octan-3-on

In Analogie zu Beispiel 1a erhält man aus 2 g (1S,5R,6R,7R)-6- [(E)-(3R, 4RS)-3-Hydroxy-4-fluor-1-octenyl] -7- benzoyloxy- 2-2-oxabicyclo [3.3.0] octan-3-on 2,3 g der Titelverbindung als Öl.

IR: 2958, 2930, 2840, 1768, 1716, 1600, 1590, 1272, 976/cm.

Beispiel 7

5-Cyano-2-descarboxy-2-hydroxymethyl-16-methyl-18,19-tetradehydroprostacyclin

In Analogie zu Beispiel 1 erhält man aus 1 g (1S, 5R, 6R, 7R)-6-[(E)-(3S, 4RS)-3-Benzoyloxy-4-methyl-1-octen -6- in-yl] -7- benzoyloxy-2-oxabicyclo [3.3.0] octan-3-on 280 mg der Titelverbindung als Öl.

IR: 3605, 3430, 2960, 2936, 2200, 1650, 976/cm.

Das Ausgangsmaterial für die obige Titelverbindung wird wie folgt hergestellt:

7a) (1S,5R,6R,7R)-6-[(E)-(3S,4RS)-3-Benzoyloxy- 4-methyl- 1-octen- 6-in-yl]-7-benzoyloxy-2-oxabicyclo[3.3.0]octan-3-on.

In Analogie zu Beispiel 1a erhält man aus 0,9 g (1S,5R,6R, 7R)-6-[(E)-(3S,4RS)-3-Hydroxy-4-methyl-1-octen-6-in-yl]-7-benzoyloxy-2-oxabicyclo[3.3.0]octan-3-on 1,04 g der Titelverbindung als Öl.

IR: 2960, 2932, 2845, 1765, 1712, 1600, 1588, 1270, 972/cm.

Beispiel 8

5-Cyano- 2-descarboxy- 2-hydroxymethyl-16-phenyl-17,18,19,20-tetranor-prostacyclin

In Analogie zu Beispiel 1 erhält man aus 1 g (1S, 5R, 6R, 7R)-6-[(E)-(3S)- 3-Benzoyloxy-4-phenyl- 1-butenyl]- 7-benzoyloxy- 2-oxabicyclo [3.3.0] octan-3-on 300 mg der Titelverbindung als Öl.

IR: 3600, 3410, 2958, 2934, 2200, 1652, 1602, 974/cm.

Das Ausgangsmaterial für die obige Titelverbindung wird wie folgt hergestellt:

8a) (1S,5R,6R,7R)-6-[(E)-(3S)-Benzoyloxy-4-Phenyl-1-butenyl]- 7-benzoyloxy-2-oxabicyclo[3.3.0]octan-3-on

In Analogie zu Beispiel 1a erhält man aus 1 g (1S, 5R, 6R, 7R)-6-[(E) -(3S)-3-Hydroxy-4-phenyl-1-butenyl]- 7-benzoyloxy- 2-oxabicyclo [3.3.0] octan-3-on 1,25 g der Titelverbindung als Öl.

IR: 2960, 2940, 2832, 1765, 1718, 1600, 1588, 1275, 974/cm.

Beispiel 9

5-Cyano- 2-descarboxy- 2-hydroxymethyl-13, 14-dihydro-16-phenoxy-17, 18, 19, 20-tetranor-prostacyclin

In Analogie zu Beispiel 1 erhält man aus 500 mg (1S,5R,6R,7R)-6-[(3R)-3-Benzoyloxy-4-phenoxy-butyl]- 7-benzoyloxy- 2-oxabicyclo [3.3.0] octan-3-on 120 mg der Titelverbindung als Öl.

IR: 3605, 3410, 2962, 2938, 2204, 1652, 1600, 1588/cm.

Das Ausgangsmaterial für die obige Titelverbindung wird wie folgt hergestellt:

9a) (1S,5R,6R,7R)-6-[(3R)-3-Benzoyloxy-4-phenoxy- butyl]- 7-benzoyloxy- 2-oxabicyclo [3.3.0] octan-3-on

In Analogie zu Beispiel 1a erhält man aus 500 mg (1S,5R,6R,7R)- 6-[(3R)-3-Hydroxy-4-phenoxy- butyl]- 7-benzoyloxy- 2-oxabicyclo [3.3.0] octan-3-on 550 mg der Titelverbindung als Öl.

IR: 2960, 2938, 1766, 1716, 1600, 1590, 1270/cm.

Beispiel 10

5-Cyano- 2-descarboxy- 2-hydroxymethyl-16, 20-dimethyl-18, 19-tetradehydro-prostacyclin

In Analogie zu Beispiel 1 erhält man aus 500 mg (1S,5R,6R,7R)-6-[(3S)-3-Benzoyloxy-4-methyl- 1-nonen- 6-in-yl]- 7-benzoyloxy-2-oxabicyclo[3.3.0]octan-3-on 100 mg der Titelverbindung als Öl.

IR: 3600, 3420, 2956, 2934, 2840, 2202, 1650, 976/cm.

Das Ausgangsmaterial für die obige Titelverbindung wird wie folgt hergestellt:

10a) (1S,5R,6R,7R)- 6-[(3S)- 3-Benzoyloxy-4-methyl- 1-nonen- 6-in-yl]- 7-benzoyloxy-2-oxabicyclo[3.3.0]octan-3-on.

In Analogie zu Beispiel 1a erhält man aus 1 g (1S,5R,6R,7R)- 6-[(3S)- 3-Hydroxy- 4-methyl-1- nonen- 6-in-yl]- 7-benzoyloxy- 2-oxabicyclo [3.3.0] octan-3-on 1,25 g der Titelverbindung als Öl.

IR: 2960, 2935, 1770, 1716, 1600, 1588, 1274, 976/cm.

Beispiel 11

(5Z)- 5-Cyano-2-descarboxy-2-hydroxymethyl- 16-phenoxy- 17,18,19,20- tetranor-prostacyclin

0,60 g (5Z)- 5-Cyano- 2-descarboxy- 2-(dimethyl- tert.- butylsilyloxymethyl- 16-phenoxy-17, 18, 19, 20-tetranor-prostacyclin (siehe Beispiel 2) rührt man 18 Stunden mit 30 ml einer Mischung aus Eisessig/Wasser/Tetrahydrofuran (65+35+10) und dampft anschliessend im Vakuum zur Trockne. Nach Chromatographie des Rückstands an Kieselgel, wobei mit Methylenchlorid/Isopropanol (9+1) eluiert wird, erhält man 0,41 g der Titelverbindung als farbloses Öl.

IR: 3605, 3400, 2930, 2865, 2210, 1650, 1600, 1588, 976/cm.

Beispiel 12

(5Z)- 5-Cyano-2-descarboxy-2-hydroxymethyl-16-methyl-prostacyclin

Zu 5,1 ml Diisopropylamin gibt man bei −25 °C 23,5 ml einer 1,53 molaren Lösung von Butyllithium in Hexan, versetzt mit 6 ml Hexamethylphosphorsäuretriamid und tropft bei −70 °C dazu eine Lösung von 8,2 g 6-(Dimethyl-tert.-butylsilyloxy)-hexannitril in 5 ml Tetrahydrofuran. Nach 30 Minuten wird eine Lösung von 4,85 g (1S,5R,6R,7R)-6-[(E)-(3S, 4RS)-3-Benzoyloxy- 4-methyl- 1-octenyl]- 7-benzoyloxy-2-oxabicyclo [3.3.0] octan-3-on in 40 ml Äther und 40 ml Tetrahydrofuran zugetropft, dann weitere 15 Minuten bei −70 °C gerührt und die Lösung auf wässrige Citronensäurelösung gegeben, wobei ein pH-Wert von 5 erreicht wird. Man extrahiert mehrmals mit Äther, schüttelt die organische Phase mit Sole, trocknet über Magnesiumsulfat, filtriert und reinigt den Eindampfrückstand mit Essigester an Kieselgel. Man erhält 3,6 g ölige Substanz, die zur Wasserabspaltung in 250 ml Äther gelöst und 1 Stunde bei 20 °C mit 1 ml 45%iger Bortrifluorid-ätherat-Lösung behandelt wird. Anschliessend giesst man auf 5%ige Natriumbicarbonatlösung, trennt die Phasen, wäscht mit Sole neutral, trocknet über Magnesiumsulfat und dampft im Vakuum ein. Nach Chromatographie des Rückstandes an Kieselgel erhält man zunächst 1,3 g (5E)-5-Cyano-2-descarboxy-2-(dimethyl- tert.- butylsilyloxymethyl)-16-methyl-prostacyclin und als polarere Komponente 1,05 g des entsprechenden (5Z)-5-Cyano-2-descarboxy- 2-(dimethyl-tert.-butylsilyloxymethyl)-16-methyl-prostacyclin.

Zur Silylätherspaltung rührt man 1,05 g der (5Z)-konfigurierten Verbindung 18 Stunden bei 20 °C mit 35 ml einer Mischung aus Eisessig/Wasser/Tetrahydrofuran (65/35/10), dampft im Vakuum ein und chromatographiert den Rückstand an Kieselgel, wobei mit Methylenchlorid/Isopropanol (9+1) eluiert wird. Man erhält 600 mg der Titelverbindung als farbloses Öl.

IR: 3610, 3410, 2958, 2936, 2862, 2200, 1650, 1601, 972/cm.

## Patentansprüche

1) Prostacyclinderivate der allgemeinen Formel I

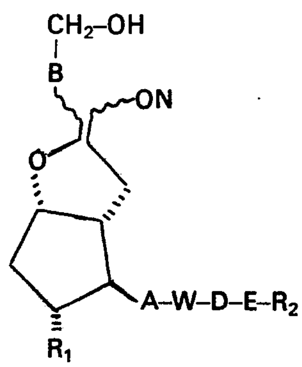

(I)

worin

B eine geradkettige oder verzweigtkettige Alkylengruppe mit 1–5 C-Atomen,

A eine $-CH_2-CH_2-$, trans-CH=CH- oder $-C\equiv C-$Gruppe,

W eine freie oder funktionell abgewandelte Hydroxymethylengruppe oder freie oder funktionell abgewandelte $-\overset{\displaystyle CH}{\underset{\displaystyle OH}{C}}-$ Gruppe, wobei die OH-Gruppe α- oder β-ständig sein kann,

D und E gemeinsam eine direkte Bindung oder

D eine geradkettige oder verzweigte gesättigte Alkylengruppe mit 1–5 C-Atomen, die gegebenenfalls durch Fluoratome substituiert sein kann,

E Sauerstoff, eine $-C\equiv C-$Bindung oder eine direkte Bindung,

$R_2$ eine Alkylgruppe mit 1–7 C-Atomen oder eine Phenylgruppe und

$R_1$ eine freie oder funktionell abgewandelte Hydroxygruppe bedeuten.

2) (5Z)- 5-Cyano- 2-descarboxy- 2-hydroxymethyl-16-phenoxy-17, 18, 19, 20-tetranor-prostacyclin

3) Verfahren zur Herstellung der Prostacyclinderivate der allgemeinen Formel I, dadurch gekennzeichnet, dass man eine Verbindung der allgemeinen Formel II

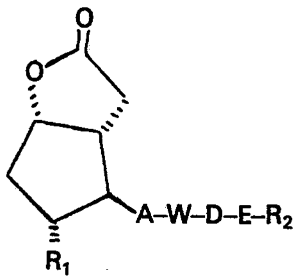

(II)

worin $R_1$, $R_2$, A, W, D, E die oben angegebenen Bedeutungen aufweisen, gegebenenfalls nach Schutz anwesender freier Hydroxygruppen nach Umsetzung mit einem aus dem Nitril der allgemeinen Formel III und Lithiumdiisopropylamid hergestellten Carbanion, wobei $R_3$ einen leicht abspaltbaren Ätherrest

$N \equiv C-CH_2-B-CH_2OR_2$ (III)

bedeutet und B die oben angegebene Bedeutung besitzt, einer Säurebehandlung unterwirft.

4) Arzneimittel, bestehend aus einer oder mehreren Verbindungen gemäss Anspruch 1 und üblichen Hilfs- und Trägerstoffen.

**Revendications**

1. Dérivés de la prostacycline qui répondent à la formule générale I:

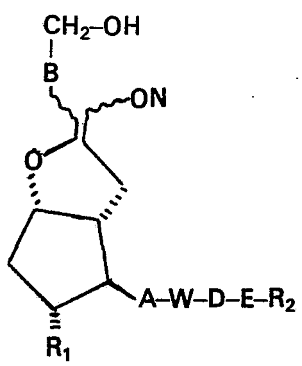

(I)

dans laquelle

B représente un radical alkylène, linéaire ou ramifié, contenant de 1 à 5 atomes de carbone,

A représente un radical $-CH_2-CH_2-$, un radical $-CH=CH-$ trans ou un radical $-C \equiv C-$,

W représente un radical hydroxyméthylène libre ou sous la forme d'un dérivé fonctionnel ou un radical $-\overset{CH_3}{\underset{OH}{C}}-$ libre ou sous la forme d'un dérivé fonctionnel, le radical $-OH$ ayant la configuration $\alpha$ ou la configuration $\beta$,

D et E forment ensemble une liaison directe ou

D représente un radical alkylène saturé, linéaire ou ramifié, qui contient de 1 à 5 atomes de carbone et qui peut porter des atomes de fluor,

E représente l'oxygène, une liaison $-C \equiv C-$ ou une liaison directe,

$R_2$ représente un radical alkyle en $C_1-C_7$ ou un radical phényle et

$R_1$ représente un radical hydroxy libre ou sous la forme d'un dérivé fonctionnel.

2. Cyano-5 décarboxy-2 hydroxyméthyl-2 phénoxy-16 tétranor-17,18,19, 20 prostacycline-(5Z).

3. Procédé de préparation de dérivés de la prostacycline répondant à la formule générale (I), procédé caractérisé en ce qu'on fait réagir un composé répondant à la formule générale II

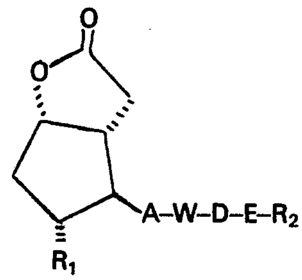

(II)

dans laquelle $R_1$, $R_2$, A, W, D et E ont les significations indiquées ci-dessus, avec un carbanion préparé à partir du diisopropylamidure de lithium et d'un nitrile répondant à la formule générale III

$N \equiv C-CH_2-B-CH_2OR_3$ (III)

dans laquelle B a la signification précédemment donnée, puis on soumet le composé obtenu, le cas échéant après en avoir protégé les éventuels radicaux hydroxy libres, à un traitement par un acide.

4. Médicament contenant un ou plusieurs composés selon la revendication 1 ainsi que des adjuvants et excipients usuels.

**Claims**

1) Prostacyclin derivatives of the general formula I

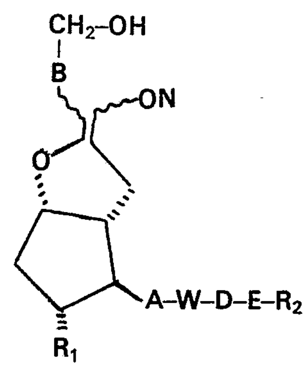

(I)

in which

B represents a straight-chain or branched-chain alkylene group having from 1 to 5 carbon atoms,

A represents a $-CH_2-CH-$, trans-$CH=CH-$ or $-C \equiv C-$ group,

W represents a free or functionally modified hydroxymethylene group or a free or functionally

modified 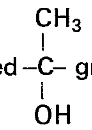 group, in which the OH group can be in the α-configuration or β-configuration,

D and E together represent a direct bond, or

D represents a straight-chain or branched saturated alkylene group having from 1 to 5 carbon atoms, which may optionally be substitued by fluorine atoms,

E represents oxygen, a $-C\equiv C-$ bond or a direct bond,

$R_2$ represents an alkyl group having from 1 to 7 carbon atoms or a phenyl group and

$R_1$ represents a free or functionally modified hydroxy group.

2) (5Z)-5-cyano-2-decarboxy-2-hydroxymethyl-16-phenoxy-17, 18, 19, 20-tetranorprostacyclin.

3) Process for the preparation of the prostacyclin derivatives of the general formula I, characterised in that a compound of the general formula II

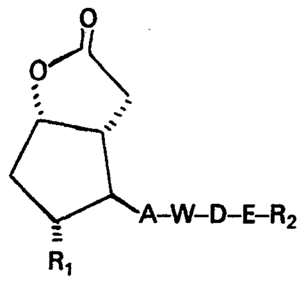

(II)

in which $R_1$, $R_2$, A, W, D and E have the above-mentioned meanings, is subjected to an acid treatment, optionally after any free hydroxy groups present have been protected, after a reaction with a carbanion which has been prepared from the nitrile of the general formula III and lithium diisopropylamide in which $R_3$ represents a readily cleavable ether radical

$$N\equiv C{-}CH_2{-}B{-}CH_2OR_3 \qquad (III)$$

and B has the above-mentioned meaning.

4) Medicament comprising one or more compounds according to claim 1 and customary auxiliaries and carriers.